(19) Europäisches Patentamt / European Patent Office / Office européen des brevets

(11) **EP 3 346 985 B1**

(12) **EUROPEAN PATENT SPECIFICATION**

(45) Date of publication and mention of the grant of the patent:
**24.08.2022 Bulletin 2022/34**

(21) Application number: **16757900.2**

(22) Date of filing: **30.08.2016**

(51) International Patent Classification (IPC):
*A61K 8/34* (2006.01)        *A61K 8/49* (2006.01)
*A61K 8/365* (2006.01)        *A61K 8/42* (2006.01)
*A61K 8/60* (2006.01)        *A61Q 19/00* (2006.01)

(52) Cooperative Patent Classification (CPC):
(C-Sets available)
**A61K 8/365; A61K 8/345; A61K 8/347; A61K 8/42;
A61K 8/4973; A61K 8/602; A61K 31/05;
A61K 31/192; A61K 31/34; A61K 31/704;
A61P 17/16; A61P 39/06; A61Q 19/00;**
A61K 2800/75                    (Cont.)

(86) International application number:
**PCT/EP2016/070375**

(87) International publication number:
**WO 2017/042049 (16.03.2017 Gazette 2017/11)**

(54) **COMPOSITION FOR TOPICAL APPLICATION COMPRISING DIMETHYL ISOSORBIDE, A POLYOL, AND A PHENOLIC OR POLYPHENOLIC ANTIOXIDANT**

ZUSAMMENSETZUNG ZUR TOPISCHEN ANWENDUNG MIT DIMETHYLISOSORBID, EINEM POLYOL UND EINEM PHENOLISCHEN ODER POLYPHENOLISCHEN ANTIOXIDATIONSMITTEL

COMPOSITION POUR APPLICATION TOPIQUE COMPRENANT DE L'ISOSORBIDE DE DIMÉTHYLE, UN POLYOL ET UN ANTIOXYDANT POLYPHÉNOLIQUE OU PHÉNOLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2015 EP 15184058**

(43) Date of publication of application:
**18.07.2018 Bulletin 2018/29**

(73) Proprietor: **MedSkin Solutions Dr. Suwelack AG
48727 Billerbeck (DE)**

(72) Inventors:
• **KUNZ, Michael
  48153 Münster (DE)**
• **KUHLMANN, Fabian
  48720 Rosendahl (DE)**
• **BEHRENS, Daniel
  48301 Nottuln (DE)**

(74) Representative: **CH Kilger Anwaltspartnerschaft mbB
Fasanenstraße 29
10719 Berlin (DE)**

(56) References cited:
**WO-A1-2009/129627     US-A- 5 824 326
US-B1- 6 632 444**

• **DATABASE GNPD [Online] MINTEL; January 2011 (2011-01), Mintel: "Ultim-age The Ultimate Anti-Ageing Serum", XP002751763, Database accession no. 1480536**
• **DATABASE GNPD [Online] MINTEL; August 2008 (2008-08), Mintel: "Face Forward Primer Age Response Formula", XP002751764, Database accession no. 956741**
• **DATABASE GNPD [Online] MINTEL; January 2008 (2008-01), Mintel: "Salicylic acid acne treatment", XP002762396, Database accession no. 850314**

(52) Cooperative Patent Classification (CPC): (Cont.)

C-Sets
**A61K 31/05, A61K 2300/00;
A61K 31/34, A61K 2300/00**

**Description**

**FIELD OF THE INVENTION**

The present invention is in the field of skin care and medical treatment of the skin. The invention relates in particular to a composition suitable for topical application comprising water, dimethyl isosorbide, a polyol, panthenol or dipotassium glycyrrhizinate and a phenolic or polyphenolic antioxidant, wherein the polyol is 1,5-pentanediol.

**BACKGROUND**

Phenolic and polyphenolic antioxidants are a class of molecules that are of high interest for skin care and the treatment of skin disorders. They show great potential for use in medical applications and cosmetic treatments due to their antioxidative characteristics. The antioxidative properties of phenols and polyphenols derive from their ability to scavenge free radicals. Many potential benefits in cosmetic and medical applications are associated with phenolic molecules. Those are for example wrinkle reduction, skin whitening/de-pigmentation, anti-inflammatory effects and protection from UV radiations. Certain phenolic and polyphenolic compounds are also thought to be useful in the treatment of skin disorders that include for example acne, herpes simplex, rosacea, actinic keratosis and psoriasis.

One polyphenol of particular interest is resveratrol (3,5,4'-trihydroxy-*trans*-stilbene). Resveratrol can for example be found in white hellebore roots and wine, in particular in red wine. It was first isolated in the early 1940s and is reported in US patent application 2002/0173472 to be highly effective in treating skin conditions associated with inflammation, skin damage associated with exposure to the sun, and the effect of natural aging.

Ferulic acid is another phenolic antioxidant that has attracted interest for its potentially beneficial properties in skin care. It can for example be found in seeds such as those of coffee, apple, artichoke, peanut, and orange, as well as in both seeds and cell walls of commelinid plants such as rice, wheat, oats, the Chinese water chestnut and pineapple.

Many phenolic antioxidants are poorly soluble in water, due to the highly hydrophobic properties of the aromatic ring. This is problematic for the use of phenolic or polyphenolic antioxidants in topical applications since they need to be dissolved in order to efficiently penetrate the skin and carry out their function. This problem can be overcome by dissolving the phenolic or polyphenolic antioxidants in compositions with a high concentration of solvent. The downside of this solution is that such compositions tend to be highly irritating for the skin.

The international patent application WO 2009/129627 proposes the use of dimethyl isosorbide or diethylene glycol monoethyl ether in the preparation of stable emulsions that comprise resveratrol. Dimethyl isosorbide is preferred because it increases the tolerability of the formulation in comparison with classic solvents.

However, even with dimethyl isosorbide in the composition at a level that is tolerable for the skin, the solubility of many phenolic or polyphenolic antioxidants remains low. In light of the prior art, there is therefore a need to find solvent combinations with better solubilising capacities for phenolic and polyphenolic antioxidants that do not cause skin irritation in order to fully unleash the many potential benefits of this class of components in skin care and the treatment of skin disorders.

**SUMMARY OF THE INVENTION**

The invention relates to a composition suitable for topical application comprising water, dimethyl isosorbide, a polyol, panthenol or dipotassium glycyrrhizinate and a phenolic or polyphenolic antioxidant, wherein the polyol is 1,5-pentanediol.

The invention also relates to a method for producing a composition according to the invention comprising the steps of

>   a) providing an aqueous solution comprising dimethyl isosorbide, panthenol or dipotassium glycyrrhizinate and a polyol , wherein the polyol is 1,5-pentanediol
>   b) providing a phenolic or polyphenolic antioxidant;
>   c) mixing the aqueous solution and the phenolic or polyphenolic antioxidant shortly before use.

The invention further relates to a kit for making a composition according the invention comprising:

>   a) an aqueous solution comprising dimethyl isosorbide, panthenol or dipotassium glycyrrhizinate and a polyol, wherein the polyol is 1,5-pentanediol
>   b) a phenolic or polyphenolic antioxidant.

In some embodiments of the invention, the phenolic or polyphenolic antioxidant is provided in the form of a lyophilisate.

The invention also relates to the non-therapeutic use of the composition as defined above for topical application.

Also part of the present disclosure is a non therapeutic method of treatment comprising the step of applying the composition according to the invention to the skin of a subject.

## DEFINITIONS

The following definitions are provided for specific terms which are used in the application. "Dimethyl isosorbide", also known by the abbreviation DMI, is a solvent with the chemical formula 1,4:3,6-Dianhydro-2,5-di-O-methyl-D-glucitol. It is available commercially for example from Croda under the trademark Arlasolve DMI. A purified version commercialized under the trademark Super Refined Arlasolve DMI is also available. Dimethyl isosorbide is a unique solubiliser, emulsifier and emollient with unusual properties. It is practically non-toxic, water soluble, oil soluble, has a high boiling point and low freezing point. Dimethyl isosorbide is miscible with many organic solvents, pH stable, non-greasy, non-drying, non-irritating, colourless, practically odourless, inert and nonvolatile. More details about dimethyl isosorbide properties can for example be found on the Grant Industries' dimethyl isosorbide application guide.

A "polyol" is used here as meaning a molecule containing multiple hydroxyl groups. "1,5-pentanediol" is a polyol of the subgroup of alkane diols. It is also known under the names pentane-1,5-diol, pentylene glycol, pentamethylene glycol, and 1,5-dihydroxypentane. It may also be referred to in this document with the abbreviation 1,5-PD.

1,3-butanediol is also a polyol of the subgroup of alkane diols. Its other names include butane-1,3-diol, 1,3-butane glycol, 1,3-dihydroxypropane. In this document it is also sometimes referred to with the abbreviation 1,3-BG.

"Panthenol" (also known as pantothenol, bepanthen and dexpanthenol) is the alcohol analogue of pantothenic acid (vitamin B5), and is thus a provitamin of B5. This molecule has been found to have anti skin irritation properties. The chemical formula of panthenol is 2,4-dihydroxy-N-(3-hydroxypropyl)-3,3-dimethylbutanamide.

"Dipotassium glycyrrhizinate" is a Dipotassium glycyrrhizinate is a compound that can for example be obtained by extraction with water from liquorice root. It is known at least under the following names: dipotassium glycyrrhizinate, dipotassium glycyrrhizate; glycyrrhizinate dipotassium, and dipotassium (3beta,20beta)-20-carboxy-11-oxo-30-norlean-12-en-3-yl-2-O-beta-D-gucopyranuronosyl-alpha-D-glucopyranosiduronate.

A "solubiliser mix" or "solubiliser complex" as used in this document refers to a mixture of solvents which is particularly apt at solubilising water-insoluble phenolic or polyphenolic antioxidants. Solubiliser mixes according to the invention comprise dimethyl isosorbide, a polyol, panthenol or dipotassium glycyrrhizinate as anti skin irritation compounds, wherein the polyol is 1,5-pentanediol. Preferably the anti skin irritation compound has the additional property of increasing the solubility of the polyphenol.

A "phenolic antioxidant" is known to the person skilled in the art as a molecule with at least one phenol function. An example of such a phenolic compound is for example ferulic acid.

A "polyphenolic compound" is known to the person skilled in the art as a molecule with more than one phenol function. Polyphenols can be flavonoid polyphenols such as epigallochatechin, gallate, epi- or gallochatechin, baicalin, taxifolin, quercetin, hesperetin or genistein. Alternatively, polyphenols can be non-flavonoid polyphenols. Resveratrol for example is a non-flavonoid polyphenol. As a note, ferulic acid though technically not a polyphenol is often classed in the scientific literature as a polyphenol and is therefore also a polyphenol by convention (see for example Manach et al., Am J Clin Nutr May 2004 vol. 79 no. 5 727-747). Ferulic acid can therefore also often be classed as a non-flavonoid polyphenol.

The phrases "phenolic or polyphenolic antioxidants" and "phenolic or polyphenolic compounds" are often used interchangeably here as most phenolic or polyphenolic compounds have antioxidant properties.

As used herein, an "antioxidant" is a substance that, when present in a mixture containing an oxidizable substrate biological molecule, significantly delays or prevents oxidation of the substrate biological molecule. Antioxidants can act by scavenging biologically important reactive free radicals or other reactive oxygen species ($.O_2$ -, $H_2O_2$, .OH, HOCl, ferryl, peroxyl, peroxynitrite, and alkoxyl), or by preventing their formation, or by catalytically converting the free radical or other reactive oxygen species to a less reactive species.

When used to describe a composition or formulation, "suitable for topical applications" means that the composition or formulation, when applied to the skin, causes minimum damage and causes minimal health risks. Particularly, suitable for topical application, in the context of this invention means, that the non-water solvent-content of the composition is minimised in order not to cause skin irritation. A composition suitable for topical application will typically comprise less than 35 % of non-water solvent. Such non-water solvents in the context of the invention are dimethyl isosorbide, polyols and anti skin irritating agents such as panthenol.

The concept of "labile active substances" refers to substances which are substantially degraded under standard storage conditions during a determined period. The determined period corresponds to the typical shelf life of a cosmetic or treatment product and should be seen as lasting up to several years, but may be as short as several months, several weeks, several days or several hours depending on the product. In contrast, "stable active substances" are those that are not significantly degraded during the same determined period and under similar conditions.

## DETAILED DESCRIPTION OF THE INVENTION

The invention relates to a composition suitable for topical application comprising water, dimethyl isosorbide, a polyol, panthenol or dipotassium glycyrrhizinate and a phenolic or polyphenolic antioxidant, wherein the polyol is 1,5-pentanediol. A method for producing a composition according to the invention is also part of the invention, as is a kit for making the composition. Optionally, the phenolic or polyphenolic antioxidant is provided in the form of a lyophilisate for the method and the kit. The non-therapeutic use of the composition according to the invention for a treatment by topical application is also within the scope of the invention.

Phenolic or polyphenolic antioxidants are thought to have many potential health benefits when applied to the skin. Resveratrol for example is thought to offer many applications such as in wrinkle reduction, antioxidative protection, skin whitening/de-pigmentation, and be an anti-inflammatory molecule. It is also thought to be useful in the treatment of skin disorders such as acne, herpes simplex, rosacea, actinic keratosis and psoriaris. Ferulic acid, a phenolic antioxidant is also expected to be effective in wrinkle reduction, as well as providing anti-irritation and antioxidative protection. In order for these polyphenols to carry out their function upon topical application, they have to be absorbed by the skin. This can only happen if they are at least partially dissolved in the composition in which they are to be applied to the skin. However, due to their often low solubility in water, of the order of less than 0.01 wt% for resveratrol and ferulic acid, it can be difficult to dissolve enough of the phenolic or polyphenolic antioxidants for them to be effective. The solubility of phenolic or polyphenolic antioxidants is generally high in certain types of pure solvents, but solutions with high non-water solvent concentrations are not well suited for topical applications. Such formulations can indeed lead to skin irritation. Even dimethyl isosorbide, which in its pure form dissolves resveratrol and ferulic acid rather well (21.4 and 15.7 wt% respectively, see example 1) is considered by the US Food and Drug Administration (FDA) not to be suitable for pharmaceutical topical applications at a concentration above 15 wt%. It is therefore an aim of the inventors to find a solvent mix that allows relatively high solubility of phenolic or polyphenolic antioxidants, but that requires the presence of only low amounts of total non-water solvents and low amounts of individual non-water solvents. This would allow to minimise the negative effects of any one solvent.

The inventors unexpectedly found that the solubility of phenolic and polyphenolic compounds in an aqueous solution is relatively high when the solution comprises dimethyl isosorbide and a polyol (see example 1). Indeed, the solubility of the phenolic or polyphenolic compounds is as high, or even higher, in a mix with the dimethyl isosorbide (10 %) and polyol (10 %) compared to the same mix with 20 % dimethyl isosorbide. This is unexpected because on their own, the polyols are generally less effective at dissolving the phenolic or polyphenolic compounds than DMI. This technical effect is useful for making topically applicable compositions comprising phenolic or polyphenolic antioxidants with a low overall non-water solvent content and a low content of the individual solvents.

One of the most important aspects of the invention is therefore to be found in a solubiliser mix with a relatively low non-water solvent concentration that nonetheless allows a relatively high solubility of phenolic or polyphenolic antioxidants and is compatible with topical application.

One technical effect obtained with the solubiliser mix is therefore a comparatively high phenolic or polyphenolic antioxidant solubilisation despite low skin irritation. This low skin irritation stems from the fact that a relatively low percentage of non-water solvents is required, and only a small amount of each individual solvent is present, thereby mitigating their individual effects. Another advantage is that since only a low percentage of non-water solvents is necessary for a relatively high phenolic or polyphenolic antioxidant dissolution, the composition can accommodate further components such as additional active substances, an oil phase, emulsifiers, polymers, preservatives or other auxiliary components which would otherwise be taken up by the solvents.

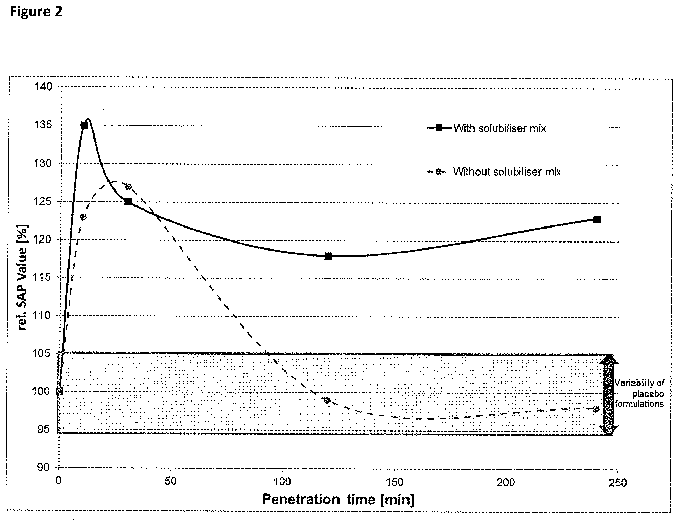

A second technical effect of the solubiliser mix is that when applied to the skin, it allows an increased phenolic or polyphenolic antioxidant-mediated antioxidative protection effect of the formulation (see example 5 and figure 2). This effect can most probably be attributed to the relatively high solubility of the antioxidant in the solubiliser mix.

A third technical effect of the solubiliser mix is that when applied to the skin, it allows increased phenolic or polyphenolic antioxidant-mediated protection against UV radiation-induced damage (see example 6 and figure 2). This effect can most probably also be attributed to the high solubility of phenolic or polyphenolic antioxidants in the solubiliser mix.

The solubiliser mix according to the invention also has the advantage that the quantities and ratios of the different solubilising components can be adjusted individually in order to optimise the skin penetration kinetics depending on the skin type.

Consequently, the present invention relates in one embodiment to a composition suitable for topical application comprising water, dimethyl isosorbide, a polyol, panthenol or dipotassium glycyrrhizinate and a phenolic or polyphenolic antioxidant, wherein the polyol is 1,5-pentanediol.

In one embodiment, the dimethyl isosorbide content of the composition according to the invention is 0.1 to 50 wt%, preferably 0.5 to 25 wt%, more preferably 2 to 20 wt%, even more preferably 5 to 15 wt%, yet more preferably from 6 to 14 wt% and most preferably 10 wt%.

The polyol comprised in the composition according to the invention is 1,5-pentanediol.

In one embodiment, the polyol content of the composition according to the invention is 0.1 to 50 wt%, preferably 0.5 to 25 wt%, more preferably 2 to 20 wt%, even more preferably 5 to 15 wt% and most preferably 10 wt%.

In one embodiment, the phenolic or polyphenolic antioxidant comprised in the composition according to the invention is a phenolic or polyphenolic antioxidant with low solubility in water. Low solubility in water is defined here as less than 10 wt%, preferably less than 1 wt%, more preferably less than 0.1 wt%, even more preferably less than 0.05 wt%.

In one embodiment, the phenolic or polyphenolic antioxidant is a non-flavonoid phenolic or polyphenolic antioxidant. Preferably the non-flavonoid phenolic or polyphenolic antioxidant is selected from the group consisting of resveratrol, and ferulic acid. In an alternative preferred embodiment, the phenolic or polyphenolic antioxidant is a polyphenol. Most preferably, the polyphenol is resveratrol.

In an alternative embodiment, the polyphenol antioxidant comprised in the composition according to the invention is a flavonoid polyphenol, preferably the flavonoid polyphenol is selected from the group consisting of epigallochatechin gallate, epi- or gallochatechin, baicalin, taxifolin, quercetin, hesperetin or genistein.

In a preferred embodiment, the phenolic or polyphenolic antioxidant content of the composition according to the invention is 0.001 to 20 wt%, preferably 0.01 to 10 %, more preferably 0.05 to 5 %, more preferably 0.1 to 4 %, more preferably 0.5 to 3.5 %, more preferably 1 to 3 %, more preferably 1.5 to 2.5 %, more preferably 2 to 2.5 %, yet more preferably 2 % and most preferably 2.4 %.

In one embodiment, the dimethyl isosorbide content of the composition according to the invention is 0.1 to 50 wt%, the polyol content is 0.1 to 50 wt%, and the phenolic or polyphenolic antioxidant content is 0.001 to 20 wt%.

In one embodiment, a composition suitable for topical application is a composition wherein the dimethyl isosorbide content is at most 15 wt%, and the polyol content is at most 15 wt%. Preferably, the dimethyl isosorbide content is at most 10 wt%, and the polyol content is at most 10 wt%. In an alternative embodiment, a composition suitable for topical application is a composition wherein the water content is at least 50 wt%, preferably at least 60 wt% or more preferably at least 70 wt%. In another embodiment, a composition suitable for topical application is a composition wherein the combined water content and oil phase content is at least 50 wt%, preferably at least 60 wt%, and more preferably at least 70 wt%. In such an embodiment, the oil phase content of the composition should be between 1 wt% and 40 wt%, preferably between 2 wt% and 20 wt%, more preferably between 3 and 15 wt%, even more preferably between 4 and 10 wt%, and most preferably 5 wt% of the composition.

In one embodiment, a composition suitable for topical application is a composition wherein the non-water solvent content is 30 wt% or less, preferably 25 wt% or less, more preferably 21 wt% or less, even more preferably 20 wt% or less and most preferably 15 % or less.In a preferred embodiment, the composition according to the invention in addition to water, dimethyl isosorbide, a polyol and a phenolic or polyphenolic antioxidant comprises an anti skin irritation compound. Preferably, the anti skin irritation compound has the property of increasing the solubility of the polyphenol in the composition according to the invention.

The anti skin irritation compound is selected from the group comprising panthenol or dipotassium glycyrrhizinate.

The inventors have surprisingly found that the anti skin irritation compounds panthenol and dipotassium glycyrrhizinate allow a synergistic increase of the solubility of phenolic and polyphenolic antioxidants when either of these compounds is present in a solubilising mix comprising water, dimethyl isosorbide and a polyol (see example 1). This effect is unexpected, as it could not have been predicted that relatively small amounts of panthenol or dipotassium glycyrrhizinate would allow a large increase in the solubility of phenolic or polyphenolic antioxidants in the presence of dimethyl isosorbide and a polyol. A clear advantage of this finding is that the overall non-water solvent concentration can be low and nonetheless allow a relatively high phenolic or polyphenolic antioxidant solubility. Furthermore, because of its higher solubility, less of the phenolic or polyphenolic antioxidant, which can itself cause skin irritation, needs to be present in the solution to achieve the same therapeutic or cosmetic effect.

In one embodiment, the most preferred anti skin irritation compounds for the composition according to the invention is panthenol. In an alternative embodiment, the most preferred anti skin irritation compounds for the composition according to the invention is dipotassium glycyrrhizinate.

In one embodiment, the content of the at least one anti skin irritation compound in the composition according to the invention is 0.01 to 20 wt%, preferably 0.1 to 10 wt%, more preferably 0.2 to 5 wt%, even more preferably 0.5 to 2 wt% and most preferably 1 wt%.

In a preferred embodiment, the composition according to the invention comprises dimethyl isosorbide, 1,5-pentanediol, panthenol and resveratrol. In a more preferred embodiment, the composition according to the invention comprises 10 wt% dimethyl isosorbide, 10 wt% 1,5-pentanediol, 1 wt% panthenol and 2 % resveratrol. In an even more preferred embodiment, the composition according to the invention comprises 10 wt% dimethyl isosorbide, 10 wt% 1,5-pentanediol, 1 wt% panthenol and 2.4 % resveratrol.

In an alternative preferred embodiment, the composition according to the invention comprises dimethyl isosorbide, 1,5-pentanediol, dipotassium glycyrrhizinate and resveratrol. In a more preferred embodiment, the composition according to the invention comprises 10 wt% dimethyl isosorbide, 10 wt% 1,5-pentanediol, 1 wt% dipotassium glycyrrhizinate and 2 % resveratrol. In an even more preferred embodiment, the composition according to the invention comprises 10 wt% dimethyl isosorbide, 10 wt% 1,5-pentanediol, 1 wt% dipotassium glycyrrhizinate and 2.4 % resveratrol.

In an alternative preferred embodiment, the composition according to the invention comprises dimethyl isosorbide, 1,5-pentanediol, panthenol and ferulic acid. In a more preferred embodiment, the composition according to the invention comprises 10 wt% dimethyl isosorbide, 10 wt% 1,5-pentanediol, 1 wt% panthenol and 2 % ferulic acid. In an even more preferred embodiment, the composition according to the invention comprises 10 wt% dimethyl isosorbide, 10 wt% 1,5-pentanediol, 1 wt% panthenol and 2.4 % ferulic acid.

In an alternative preferred embodiment, the composition according to the invention comprises dimethyl isosorbide, 1,5-pentanediol, dipotassium glycyrrhizinate and ferulic acid. In a more preferred embodiment, the composition according to the invention comprises 10 wt% dimethyl isosorbide, 10 wt% 1,5-pentanediol, 1 wt% dipotassium glycyrrhizinate and 2 % ferulic acid. In an even more preferred embodiment, the composition according to the invention comprises 10 wt% dimethyl isosorbide, 10 wt% 1,5-pentanediol, 1 wt% dipotassium glycyrrhizinate and 2.4 % ferulic acid.

In one embodiment, the amounts of water, dimethyl isosorbide, polyol and/or anti skin irritation compound are adjusted according to the skin type to which it is intended to be applied. The preferred embodiments of the previous four paragraphs are particularly suited for topical application onto normal skin. However, for topical application onto sensitive skin, the percentages of non-water solvents, as well as those of the phenolic and/or polyphenolic compounds should be reduced by up to 50 %, preferably by up to 60 % and more preferably by up to 70 %.

In one embodiment, the composition according to the invention in addition to dimethyl isosorbide, a polyol, panthenol or dipotassium glycyrrhizinate and a phenolic or polyphenolic antioxidant, wherein the polyol is 1,5-pentanediol comprises at least one further compound selected from the group comprising a stable active substance, an oil phase, an emulsifier, a polymer, and a preservative system.

In one embodiment, the composition of the invention also comprises an oil phase.

In a preferred embodiment, the oil phase comprised in the composition according to the invention comprises at least one emollient compound. Preferred emollient compounds are triglycerides, fatty acid esters, fatty alcohols, naturally occurring fatty acid esters, and butters. In a more preferred embodiment, the at least one emollient compound is selected from the group comprising triglycerides from plant origin, the fatty acid esters stearates, isostearates, hydroxystearates, palmitates, ,myristates, adipates, oleates, cocoates, succinates, ethylhexanoates, the fatty alcohols cetyl, cetearyl, stearyl, oleyl, isostearyl, octyl, the naturally occurring fatty acid esters Simmondsia Chinensis (Jojoba) seed oil, and the butters Butyrospermum Parkii (shea) butter and Theobroma Cacao (Cocoa) seed butter. In a preferred embodiment, the oil phase comprises an oil phase comprising cyprylic/capric triglyceride and shea butter.

In one embodiment, the oil phase of the composition according to the invention represents 0.5 to 30 wt%, preferably 1 to 20 wt%, more preferably 2 to 10 wt%, even more preferably 3 to 8 wt%, yet more preferably 4 to 6 wt%, and most preferably 5 % of the composition.

In an alternative embodiment, the oil phase of the composition according to the invention represents 0.5 to 30 wt%, preferably 1 to 25 wt%, more preferably 2 to 23 wt%, even more preferably 5 to 20 wt%, yet more preferably 10 to 15 wt%, and most preferably 13 % of the composition.

Preferably, the composition of the invention is an emulsion.

In one embodiment, the composition according to the invention in addition comprises at least one emulsifier.

In a preferred embodiment, the at least one emulsifier comprised in the composition according to the invention is selected from the group comprising non-ionic, cationic or anionic emulsifiers, synthetic, semi-synthetic or nature-based monomeric emulsifiers, polymeric emulsifiers or emulsifying polymers. More preferably the at least one emulsifier is a PEG/PPG-free emulsifier, a low to high molecular weight emulsifier or an emulsifying polymer. The person skilled in the art will know that the amount of emulsifier necessary in an emulsion depends on the type of emulsifier. In one embodiment, the emulsifier content in the composition according to the invention is between 0.2 and 15 wt%, more preferably between 0.5 and 8 wt%, even more preferably between 1 and 5 wt%, and most preferably the emulsifier content is 1.5 wt%.

As will be known to the person skilled in the art, the visco-elastic properties of a composition can be influenced by addition of a polymer. It can therefore be advantageous to add such a polymer to a composition in order to obtain the desired visco-elastic properties.

In one embodiment therefore, the composition according to the invention in addition comprises at least one polymer. Preferably, this at least one polymer is selected from the group comprising hydrophilic, amphiphilic or hydrophobic polymers. As will be known to the person skilled in the art, hyaluronic acid has the added benefit of maintaining skin moisture after topical application to enable prolonged penetration. Therefore, the at least one additional polymer is preferably hyaluronic acid.

The person skilled in the art will also know that the addition of such a polymer also enables a shorter reconstitution time of a lyophilisate by enabling a better wettability. This is relevant for the embodiments of the invention in which the at least one phenolic or polyphenolic antioxidant is provided in the form of a lyophilisate.

In a preferred embodiment, the at least one additional polymer is present in the composition according to the invention in an amount of 0.01 to 5 wt%, preferably 0.05 to 2 wt%, more preferably 0.1 to 1 wt%, and most preferably 0.3 wt%.

In one embodiment, the composition according to the invention in addition comprises at least one preservative or preservative system.

In a preferred embodiment, the at least one preservative or preservative system comprised in the composition according to the invention is selected from the group comprising classical cosmetic preservatives and preservative systems. Preferably, the preservative or preservative system is paraben-free and/or non-formaldehyde releasing such as phenoxyethanol, benzoic acid and its derivatives, dehydroacetic acid and its derivatives, sorbic acid and its derivatives, salicylic acid and its derivatives, skin conditioning agents with antimicrobial function such as levulinic acid and its derivatives or lactobacillus ferment (filtrate), masking agents such as p-anisic acid and its derivatives, humectants with antimicrobial action such as caprylyl glycol, 1,2-hexanediol, propylene glycole, chelating agents with microbial action such as caprylhydroxamic acid or antimicrobial biofermentative extracts such as Leuconostoc/radish root ferment filtrate. In more preferred embodiment, the at least one preservative or preservative system comprised in the composition according to the invention is caprylhydroxamic acid alone or in combination with humectants with antimicrobial action, levulinic acid alone or in combination with humectants with antimicrobial action or antimicrobial extracts like lactobacillus ferment (filtrate) or Leuconostoc/radish root ferment filtrate.

In a further preferred embodiment, the preservative or preservative system is present in the composition according to the invention in an amount of 0.1 to 10 wt%, preferably 0.5 to 5 wt%, more preferably 1 to 3 wt%, and most preferably 2 wt%.

In an alternative preferred embodiment, the preservative or preservative system is present in the composition according to the invention in an amount of 0.1 to 10 wt%, preferably 0.2 to 5 wt%, more preferably 0.3 to 2 wt%, and most preferably 0.5 wt%.

In one embodiment, the composition according to the invention in addition comprises at least one auxiliary component.

In a preferred embodiment, the at least one auxiliary component comprised in the composition according to the invention is selected from the group of fragrances, buffers, pH modifiers, colouring components such as dyes or pigments, thickeners, sensorial additives that modify the haptics of the composition, sun protection factors and sensorial modifiers. More preferably, the at least one auxiliary component comprised in the composition is a thickener of natural, semi-synthetic or synthetic origin selected from the group comprising xanthan gum, polysaccharides, and carbomers. As the person skilled in the art will know, dimethyl isosorbide has a relatively strong odour, which may be unpleasant when applied to the skin. This strong odour can be masked by the addition of one or several fragrances to the composition. Therefore, in another more preferred embodiment, the at least one auxiliary component comprised in the composition is a fragrance.

In a further preferred embodiment, the at least one auxiliary compound is present in the composition according to the invention in an amount of 0.01 to 2 wt%, preferably 0.05 to 1 wt%, more preferably 0.1 to 0.5 wt%, and most preferably 0.2 wt%. And most preferably, the auxiliary compound is a perfume.

In an alternative preferred embodiment, the at least one auxiliary compound is present in the composition according to the invention in an amount of 0.05 to 2 wt%, preferably 0.1 to 1 wt%, more preferably 0.2 to 0.5 wt%, and most preferably 0.3 wt%.

In one embodiment, the composition according to the invention comprises at least one further active substance. The further active substance comprised in the composition according to the invention can be a stable active substance or a labile active substance.

In one embodiment, the at least one further stable active substance comprised in the composition according to the invention is selected from the group comprising natural, semi-synthetic or synthetic peptides, non-substituted, acetylated or fatty acid derivatives of amino acids, di-tri-or oligopeptides, aqueous or glycolic plant extracts, and moisturising components selected from the group comprising natural moisturising factors, $\alpha$-hydroxy acids and keratolytic agents.

In one embodiment, the at least one further stable active substance is present in the composition according to the invention in an amount of 0.05 to 5 wt%, preferably 0.1 to 1 wt%, more preferably 0.2 to 0.5 wt%, and most preferably 0.3 wt%. As will be clear to the person skilled in the art, the amount of the at least one further stable active substance depends on the selected active substance. Thus if the at least one further stable active substance is a non-substituted, acetylated or fatty acid derivative of an amino acid, the amount of this further stable active substance in the composition should be 1 % wt or less. In contrast, if the at least one further stable active substance is a natural moisturising factor, an $\alpha$-hydroxy acid or a keratolytic agent, the amount of further stable active substance present in the composition should be 5 wt% or less. Non-substituted, acetylated or fatty acid derivative of an amino acid, natural moisturising factors, $\alpha$-hydroxy acids and keratolytic agents are preferred further stable active substances.

Natural peptides can be obtained for example by hydrolysing proteins from various natural origins such as rice, cotton, wheat, soy, silk, or any other appropriate source of hydrolysable proteins.

In one embodiment, the at least one further labile active substance comprised in the composition according to the invention is selected from the group comprising, or preferably consisting of, a further phenolic or polyphenolic antioxidant, ascorbic acid and its derivatives, tocopherol and its derivatives, retinol or retinoic acid derivatives and enzymes such as superoxide dismutase. Preferably, the additional labile active substance is ascorbic acid.

In one embodiment, the at least one auxiliary compound is present in the composition according to the invention in an amount of 0.05 to 20 wt%, preferably 0.1 to 10 wt%, more preferably 0.2 to 5 wt%, and most preferably 3 wt%. As will be clear to the person skilled in the art, the amount of the at least one auxiliary compound present in the composition will depend on the selected auxiliary compound. If a polyphenol such as baicalin is selected, the amount in the composition should be 2 wt% or less, if ascorbic acid or one of its derivatives is selected, the amount should be 20 % or less, if tocopherol or one of its derivatives is selected, the amount should be 5 wt% or less, if retinol or a retinoic acid derivative is selected, the amount should be 5 wt% or less, and if an enzyme is selected, the amount should be 1 wt% or less.

The person skilled in the art will know that some phenolic or polyphenolic antioxidants are unstable under standard storage conditions and may therefore be degraded before they are to be used thereby reducing the shelf life of a phenolic or polyphenolic antioxidant-comprising composition. This degradation can be accelerated if the phenolic or polyphenolic antioxidant is stored in a solution. In order to overcome this problem, it is possible to store the phenolic or polyphenolic antioxidant separately from the solubilising mix (i.e. the solution that comprises dimethyl isosorbide and a polyol). The invention therefore also encompasses the composition according to the invention, wherein the water, the dimethyl isosorbide and the polyol are provided separately from the phenolic or polyphenolic antioxidant as two distinct components of the composition and the two components are mixed together shortly before use. The invention also relates to a method for producing a composition according to the invention, as well as a kit that comprises the separate components, which upon mixing will result in a composition according to the invention.

In a preferred embodiment, if the composition according to the invention is provided as two distinct components, and comprises at least one additional compound selected from the group comprising an anti skin irritation compound such as panthenol or dipotassium glycyrrhizinate, a stable active substance, a preservative or a preservative system, this at least one additional compound is provided in the component that also comprises the dimethyl isosorbide and the polyol.

One embodiment of the invention is a method for producing a composition according to the invention comprising the steps of

a) providing an aqueous solution comprising dimethyl isosorbide,panthenol or dipotassium glycyrrhizinate and a polyol , wherein the polyol is 1,5-pentanediol;
b) providing a phenolic or polyphenolic antioxidant;
c) mixing the aqueous solution and the phenolic or polyphenolic antioxidant shortly before use.

The concept of "shortly before use" should be understood as meaning days, hours or minutes before use, more preferable hours or minutes before use, and most preferably minutes before use. The use is to be understood as the topical application of the composition.

One embodiment of the invention is a kit for making a composition according to the invention comprising

a) an aqueous solution comprising dimethyl isosorbide, panthenol or dipotassium glycyrrhizinate and a polyol, wherein the polyol is 1,5-pentanediol;
b) a phenolic or polyphenolic antioxidant.

In certain circumstances it may be advantageous to provide the phenolic or polyphenolic antioxidant in the form of a lyophilisate to be mixed with the solubiliser mix (i.e. the solution comprising dimethyl isosorbide and a polyol) shortly before use. Accordingly, in one embodiment of the method according to the invention, the phenolic or polyphenolic antioxidant is provided in the form of a lyophilisate. Similarly, in one embodiment of the kit, the phenolic or polyphenolic antioxidant of the kit is a phenolic or polyphenolic antioxidant in the form of a lyophilisate.

Lyophilisation, or freeze-drying, is a technic known to the person skilled in the art. The person skilled in the art is therefore able to determine appropriate lyophilisation conditions for a phenolic or polyphenolic antioxidant -comprising substance.

In one embodiment, the method and kit according to the invention respectively provide or comprise in addition to dimethyl isosorbide, a polyol, panthenol or dipotassium glycyrrhizinate and a phenolic or polyphenolic antioxidant, wherein the polyol is 1,5-pentanediol, at least one compound selected from the group comprising an additional labile active substance, an oil phase, an emulsifier, and a polymer. Preferably, if the phenolic or polyphenolic antioxidant is to be provided/present in the method/kit according to the invention in the form of a lyophilisate, this at least one additional compound is comprised in the lyophilisate along with the phenolic or polyphenolic antioxidant.

A further advantage of providing the phenolic or polyphenolic antioxidant in lyophilised form is therefore that one or several further labile active substances can be lyophilised along with the phenolic or polyphenolic antioxidant and thereby also enjoy prolonged stability due to the lyophilisation.

Hence, in one embodiment of the invention the lyophilisate, in addition to a phenolic or polyphenolic antioxidant, comprises at least one further labile active substance. In a preferred embodiment, the at least one further labile substance is ascorbic acid or one of its derivatives, tocopherol or one of its derivatives, retinol or a retinoic acid derivative, an enzyme, preferably superoxide dismutase. In a more preferred embodiment, the at least one further labile substance is ascorbic acid.

In one embodiment, the lyophilisate that comprises the polyphenol in addition comprises at least one compound selected from the group comprising a further polyphenol, an oil phase, an emulsifier, and a polymer. In a preferred embodiment, if a further polyphenol, an oil phase, an emulsifier, or a polymer is provided in the method and/or in the kit according to the invention, this compound is provided in the lyophilisate that also comprises the phenolic or polyphenolic antioxidant.

The kit of the application is by no means limited to the components enumerated above. In one embodiment, the kit in addition to an aqueous solution and a polyol comprises at least one element selected from the group comprising instructions for the use of the kit, a container for mixing the at least two elements of the kit, a cleaning composition for removing the composition from the skin after its application, a soothing composition for treating irritated skin, a face mask, and protective gear such as eye patches.

All the embodiments that refer to the composition according to the present invention also apply to the method as disclosed in the present disclosure and the kit according to the invention. As way of example, a method for preparing a composition according to the invention comprising dimethyl isosorbide, 1,5-pentanediol, panthenol and resveratrol, would provide all these compounds necessary to make the composition. The different compounds can be provided separately from the aqueous phase and from the phenolic or polyphenolic antioxidant, or pre-mixed with one of them. Similarly, the kit according to the invention can comprise all the compounds necessary to make a given composition according to the invention.

A further embodiment of the invention is the use of a composition comprising water, dimethyl isosorbide, a polyol, panthenol or dipotassium glycyrrhizinate and a phenolic or polyphenolic antioxidant, wherein the polyol is 1,5-pentanediol, for topical application.

All preferred embodiments of the composition, the method and the kit of the invention also apply to the use of the composition. Concretely this means that the invention encompasses the use of all the compositions of the invention.

In one embodiment, the non-therapeutic use of the composition according to the invention is for wrinkle reduction, for providing an antioxidative effect (radial protection), or for skin whitening/depigmentation.

A further embodiment of the application is a non-therapeutic method of treatment comprising the step of applying the composition according to the invention to the skin of a subject. In a preferred embodiment, the subject is a mammal, preferably a human.

## EXAMPLES

The following examples are illustrative of the applicability of the present invention and are not intended to limit its scope.

Example 1

**Phenolic and polyphenolic antioxidant solubility experiments**

In their endeavour to find suitable solutions to solubilise phenolic and polyphenolic antioxidants, with low amounts of each individual solvent the inventors tested the solubility of resveratrol and ferulic acid in pure solvents and in different mixes of water and solvents (hereafter called solubiliser mixes, mixtures or complexes)

Concretely, 10 ml of a pure solvent or a solubiliser mixture were stirred in a beaker at ambient temperature. An amount of 2 mg phenolic or polyphenolic antioxidant was dispersed in the mixture and stirred. If the mixture became clear, the addition of phenolic or polyphenolic antioxidant was repeated until the mixture remained turbid even after stirring for 10 min. The maximum solubility was calculated as a percentage by mass (wt%) based on this experiment.

The results for the solubility of resveratrol and ferulic acid in pure solvent (75 wt% for panthenol) are shown in table 1.

Table 1:

| Solvent | Resveratrol solubility in pure solvent [wt%] | Ferulic acid solubility in pure solvent [wt%] |
|---|---|---|
| Water | 0.006 | 0.002 |
| 1,5-pentyleneglycol (1,5-PD) | 12.5 | 9.3 |
| 1,2-propanediol | 21.5 | n.d. |
| 1,3-propanediol | 7.2 | 6.5 |
| 1,3-butyleneglycol (1,3-BG) | 4.2 | 7.4 |
| Dimethyl isosorbide | 21.4 | 15.7 |
| Panthenol (75%) | 0.8 | n.d. |

As can be seen from these experiments, the solubility of resveratrol and ferulic acid are extremely low in water.

The solubility of resveratrol and ferulic acid was also tested in a 10 wt% solvent solution (7.5 wt% for panthenol) and the results are presented in table 2.

Table 2:

| Solvent | Resveratrol solubility in diluted solvent [wt%] | Ferulic acid solubility in diluted solvent [wt%] |
|---|---|---|
| 1,5-pentyleneglycol (1,5-PD) | **0.05** | 0.24 |
| 1,2-propanediol | 0.05 | 0.1 |
| 1,3-propanediol | 0.02 | 0.08 |
| 1,3-butyleneglycol (1,3-BG) | 0.05 | 0.1 |
| Dimethyl isosorbide | **0.05** | 0.2 |
| Panthenol (7.5%) | - | 0.16 |

From this table it is evident that the solubility of resveratrol and ferulic acid is also very low in a solution with a low solvent content.

Surprisingly however, the inventors found that a combination of low amounts of dimethyl isosorbide and a polyol allows obtaining relatively high solubility of the phenolic and polyphenolic antioxidants. This can be seen in table 3 for resveratrol and table 4 for ferulic acid. This effect is advantageous because only low amounts of each individual solvent need to be present in the solution to provide an acceptable solubility of polyphenol. When such a composition is used for topical application, this allows reducing the undesirable effects of individual solvents.

The inventors also surprisingly found that the addition of a relatively low amount of panthenol or alternatively dipotassium glycyrrhizinate to a water/dimethyl isosorbide/polyol mixture allows a large increase in phenolic or polyphenolic antioxidant solubility. For example, the addition of only 1 wt% of panthenol increases the solubility of resveratrol in a 10 wt% dimethyl isosorbide and 10 wt% 1,5-pentanediol from 0.24 wt% to 0.38 wt%.

Table 3:

| wt% DMI | wt% 1,5-PD | wt% 1,3-BG | wt% panthenol | wt-% dipotassium glycyrrhizinate | wt% resveratrol in solution |
|---|---|---|---|---|---|
| - | - | - | - | - | 0.006 |
| 10 | - | - | - | - | 0.05 |
| 20 | - | - | - | - | 0.23 |
| - | 10 | - | - | - | 0.05 |

(continued)

| wt% DMI | wt% 1,5-PD | wt% 1,3-BG | wt% panthenol | wt-% dipotassium glycyrrhizinate | wt% resveratrol in solution |
|---|---|---|---|---|---|
| - | - | - | 10/5/1 | - | 0/0/0 |
| 10 | 10 | - | - | - | 0.24 |
| 10 | - | - | 1 | - | 0.05 |
| - | 10 | - | 1 | - | 0.05 |
| 10 | 10 | - | 1/5 | - | 0.38/0.36 |
| 10 | - | 10 | 1 | - | 0.19 |
| 10 | 10 | - | - | 1 | 0.30 |
| 10 | - | 10 | - | 1 | 0.215 |

Table 4:

| wt% DMI | wt% 1,5-PD | wt% 1,3-BG | wt% panthenol | wt-% dipotassium glycyrrhizinate | wt% ferulic acid in solution |
|---|---|---|---|---|---|
| - | - | - | - | | 0.002 |
| 10 | - | - | - | | 0.2 |
| 20 | - | - | - | | 0.59 |
| - | 10 | - | - | | 0.24 |
| - | - | - | 10 | | 0.16 |
| 10 | 10 | - | - | | 0.69 |
| 10 | - | - | 1 | | 0.2 |
| - | 10 | - | 1 | | 0.17 |
| 10 | 10 | - | 1/5 | | 0.8/0.92 |
| 10 | - | 10 | - | | 0.44 |
| - | - | 10 | 1 | | 0.15 |
| 10 | - | 10 | 1/5 | | 0.41/0.57 |
| 10 | 10 | - | - | 1 | 0.905 |
| 10 | - | 10 | - | 1 | 0.675 |

Example 2

**Preparation of emulsions containing a phenolic or polyphenolic antioxidant with or without a solubilising mix**

In order to assess whether the increased solubility of the polyphenol in a solubiliser mix translates into higher activity of the phenolic or polyphenolic antioxidant, emulsions comprising a phenolic or polyphenolic antioxidant (in this case ferulic acid or resveratrol) with and without a solubiliser mix were prepared. The final concentrations of the solvents in these emulsion are 10 wt% dimethyl isosorbide, 10 wt% 1,5-pentanediol and 1 wt% panthenol.

Emulsion with ferulic acid or resveratrol, <u>without</u> the solubiliser mix:

- 900 mg of high molecular weight hyaluronic acid (GfN/Contipro 3010, 1.5 MDa) was dissolved in 247 g of distilled water, heated to 80°C and stirred by means of a 1 l vacuum mixing device at 1400 rpm and ambient pressure for 15 minutes.
- 4.5 g Sepinov EMT-10 (INCI name: Hydroxyethyl acrylate (and) Sodium Acryloyl Dimethyl Taurate Copolymer) were added and the mixture was stirred at 1400 rpm/200 mbar for further 15 minutes at 80°C.

- 7,2 g of Resveratrol (Polygonum Extract 98%, DKSH) (alternatively 7,2 g Ferulic Acid, 98%, Merck) were added and the mixture was stirred at 1400 rpm/200 mbar for further 15 minutes at 80°C.
- 9,0 g shea butter (HallStar) were dissolved in 30 g of medium chain triglyciderides (MCTs) at 80°C and the mixture was added and homogenized at 2100 rpm/200mbar for 5 minutes.
- The mixture was cooled below 40°C and 1,5 g of Spectrastat OL (INCI name: Caprylhydroxamic Acid, Caprylyl Glycol, Propanediol, Inolex® Incorporated) were added as a preservative compound and mixed for 5 min at 1400 rpm/200 mbar.
- The viscosity was measured after overnight storage of the emulsion with a Malvern Kinexus Rheometer (Plate/Plate, 40mm, 25°C, 0,2mm gap, 0,01 to 1000 1/s). The resulting emulsions had viscosities of 43.23 Pa*s (with Resveratrol) and 38.91 Pa*s (with Ferulic Acid) at 1 1/s.

Emulsion with resveratrol and with the solubiliser mix:

- 1.8 g of high molecular weight hyaluronic acid (GfN/Contipro 3010, 1.5 MDa) were dissolved in 367,8 g of distilled water, heated to 80°C and stirred by means of a 1l vacuum mixing device at 1400 rpm and ambient pressure for 15 minutes.
- 14,4 g of Resveratrol (Polygonum Extract 98%, DKSH) were dispersed in a mixture of 60g 1,5-pentandiol (98%, Merck), 60 g dimethyl isosorbide (Gransolve DMI, Grant Industries), and 6 g panthenol (75%, BASF) at room temperature for 30 minutes. The mixture was combined with the aqueous hyaluronic acid mixture and stirred at 1400 rpm/200 mbar for 15 minutes at 80°C.
- 9 g Sepinov EMT-10 (INCI name: Hydroxyethyl acrylate (and) Sodium Acryloyl Dimethyl Taurate Copolymer) were added and the mixture was stirred at 1400 rpm/200 mbar for a further 15 minutes at 80°C.
- 18 g shea butter (HallStar) were dissolved in 60 g of medium chain triglyciderides (MCTs) at 80°C and the mixture was added and homogenized at 2100 rpm/200mbar for 5 minutes.
- The mixture was cooled below 40°C and 3 g of Spectrastat OL (INCI name: Caprylhydroxamic Acid, Caprylyl Glycol, Propanediol, Inolex® Incorporated) were added as preservative compound and mixed for 5 min at 1400 rpm/200 mbar.
- The viscosity was measured after overnight storage of the emulsion with a Malvern Kinexus Rheometer (Plate/Plate, 40mm, 25°C, 0,2mm gap, 0,01 to 1000 1/s). The resulting emulsion had a viscosity of 11.1 Pa*s at 1 1/s.

By way of example, an emulsion comprising further labile compounds (in this case vitamin C, retinol, tocopherol and baicalin) but no solubiliser mix can be made as follows:

- 900mg of high molecular weight hyaluronic acid (GfN/Contipro 3010, 1.5 MDa) was dissolved in 236,7 of distilled water, heated to 80°C and stirred by means of a 1 l vacuum mixing device at 1400 rpm and ambient pressure for 15 minutes
- 4.5g Sepinov EMT-10 (INCI name: Hydroxyethyl acrylate (and) Sodium Acryloyl Dimethyl Taurate Copolymer) was added and the mixture was stirred at 1400 rpm/200 mbar for further 15 minutes at 80°C
- 9,0 g shea butter (HallStar), 3 g of tocopherol (97%, Alpha Aesar) and 6 g of retinol (98%, Sigma Aldrich) was dissolved in 30 g of medium chain triglyciderides (MCTs) at 80°C and the mixture was added and homogenized at 2100 rpm/200mbar for 5 minutes
- The mixture was cooled below 40°C and 3,0 g of Spectrastat OL (INCI name: Caprylhydroxamic Acid, Caprylyl Glycol, Propanediol, Inolex® Incorporated) was added as preservative compound and mixed for 5 min at 1400 rpm/200 mbar
- 7,2 g of Ascorbic acid (99%, DSM), 7,2 g of Resveratrol (Polygonum Extract 98%, DKSH) and 1.5 g of Baicalin (98%, Provital) was added and the mixture and was stirred at 1400 rpm/200 mbar for further 15 minutes
- The viscosity was measured after overnight storage of the emulsion with a Malvern Kinexus Rheometer (Plate/Plate, 40mm, 25°C, 0,2mm gap, 0,01 to 1000 1/s). The resulting emulsion had a viscosity of 15.4 Pa*s at 1 1/s.

Such an emulsion can be lyophilised as in example 4.

Example 3

**Emulsion stability testing**

The stability of the emulsions is thermally tested using a programmable climate chamber (Binder KBF 240) performing 24 hour climate changes from -5°C/0% relative humidity to 40°C/75% relative humidity.
Samples were transferred into 10 ml glass vials. Emulsions were tested for 30 days and visually inspected daily. All emulsion systems described in example 1 and two remained visually stable for the 30 days of the trial.

Example 4

**Lyophilisation and reconstitution of resveratrol-containing emulsions**

The inventors tested whether it would be possible to lyophilise and reconstitute a resveratrol-containing emulsion.

For lyophilisation, 7.5 ml of resveratrol-containing emulsion without solubiliser mix from example 2 were dispensed into 10 ml glass vials. Samples were frozen at -20°C overnight and placed in a Christ Epsilon 2-10D LSC plus HT device and processed for approximately 20 hours at maximum 120°C.

For further testing, lyophilisates thus obtained were reconstituted with 7.5 ml reconstitution liquid containing a solubiliser mix.

The solubiliser mix was prepared as follows:

- 2.5 g Spectrastat E (Inolex: caprylhydroxamic acid (and) ethylhexylglycerin (and) methylpropanediol) as model preservative compound were dispersed in 379 g distilled water at room temperature.
- 12.5 g panthenol (75%, BASF), 50 g dimethyl isosorbide (Gransolve DMI, Grant Industries) and 50 g 1,5-pentanediol (98%, Merck) were added. (Further aqueous solution based ingredient solutions can be added subsequently.)

The emulsion was transferred into a volumetric flask. Methanol/water 50% v/v was added as eluent and the samples were placed in an ultrasound bath for 15 min at 25°C. Samples were transferred into an HPLC vial and measured with the HPLC device (Shimadzu). HPLC analysis showed that neither a loss in resveratrol concentration nor a partial isomerisation from bioactive trans- to cis-resveratrol had occurred. Furthermore, the stability of the reconstituted emulsion was tested for 30 days according to example 3. The emulsions remained stable during the entire trial period.

In addition, the antioxidative power of emulsions with or without solubiliser mix (the emulsions of example 2), including a lyophilised emulsion that was reconstituted after lyophilisation, was measured. The lyophilisate was reconstituted and the antioxidative power was measured 30 minutes after reconstitution.

The so called antioxidative power (AP) method is based on ESR spectroscopy and allows determining the antioxidative power of active substances in vitro (Jung K, Richter J, Kabrodt K, Lucke IM, Schellenberg I, HerrlingT. The antioxidative power AP--A new quantitative time dependent (2D) parameter for the determination of the antioxidant capacity and reactivity of different plants. Spectrochim Acta A Mol Biomol Spectrosc. 63(2006):846-50; Jung K, Sacher M, Blume G, Janßen F, Herrling T. How Active are Biocosmetic Ingredients? SOFW-Journal 133 1/2 - 2007).

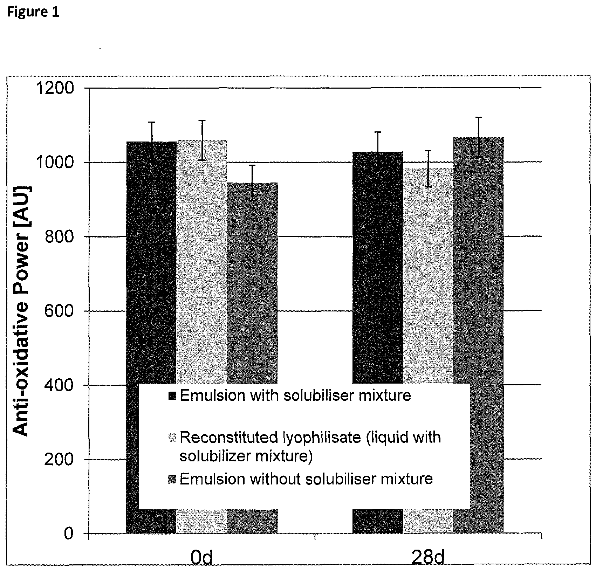

The results of this experiment are shown in figure 1. They show that the lyophilisation and subsequent reconstitution of the emulsion does not decrease the antioxidative power of the emulsion.

The experiments shown in this example demonstrate that lyophilisation of the phenolic or polyphenolic antioxidant and subsequent reconstitution of the emulsion with the solubiliser mix according to the invention do not lead to degradation of the phenolic or polyphenolic antioxidant. The phenolic or polyphenolic compound of the emulsion therefore surprisingly retains its full antioxidative properties even after lyophilisation and reconstitution. Lyophilisation therefore offers the possibility of preserving labile components in a lyophilised form before reconstituting the emulsion shortly before use.

Example 5

**The solubiliser mix according to the invention leads to better antioxidative protection of the skin.**

The inventors tested whether the higher solubility of the phenolic or polyphenolic antioxidants in a solubiliser mix according to the invention (example 1) translates into higher antioxidative protection of the skin.

Skin has intrinsic antioxidative protection thanks to the many enzymatic and non-enzymatic antioxidant systems present in the epidermis. A Skin Antioxidative Potential (SAP) can be measured by ESR spectroscopy using an active test radical that is reduced by the antioxidant systems inside the epidermis and dermis. The SAP value is a quantitative ex vivo determination of the antioxidant activity inside the epidermal and dermal layers of the skin.

A SAP value above 100% indicates that the topically applied active substance(s) was able to penetrate into the skin and is effective against free radical injury in the skin. In contrast, if the SAP value is below 100 %, the topical treatment has diminished the skin's antioxidant defense system. Topical treatments with such negative effects on the skin antioxidant system are for example application of a chemical such as detergents or physical stress such as sun exposure.

The method entails applying the composition comprising the active substance (in this case resveratrol) to pig skin biopsies and testing the antioxidative power of the active substance. The antioxidative effect is dependent on whether the active substance is absorbed into the skin.

The measurements mere carried out as follows. Skin biopsies obtained from local slaughterhouse (pig skin, 6 month old animals, external lobe of the ears) were washed, the subdermal fat was removed and the skin was cut in 2x2 cm pieces.

Emulsions made as in example 2, with or without resveratrol, and with or without a solubiliser mix (dimethyl isosorbide, 1,5-pentanediol and panthenol) were applied on the epidermal layer of the biopsies. Skin pieces were placed on a filter paper saturated with the test radical (TEMPO, 2,2,6,6-tetramethyl piperidine-N-oxyl, Sigma-Aldrich, Munich, Germany). Skin biopsies of 4 cm were taken, placed in a special ESR tissue cell and ESR spectra of the test radical were recorded in a time-course experiment.

The amplitudes of the ESR spectra were plotted against time and the obtained kinetics were fitted using a mono-exponential first order decay algorithm. The kinetic parameter k of the skin sample treated with an emulsion that does not contain resveratrol (placebo-treated) was set as 100%.

The measurements were made after penetration times of between ten minutes and four hours. All values were normalized to the placebo-treated skin. The results of the experiment are exposed in table 5 and are graphically represented for the emulsions with resveratrol, with or without the solubiliser mix, in figure 2.

Table 5:

| Penetration time [min] | Emulsion 1 | Placebo 1 | Emulsion 2 | Placebo 2 | 1% tocopherol (internal std.) |
|---|---|---|---|---|---|
| Resveratrol content | 2% | - | 2% | - | - |
| Solubiliser mix | DMI, 1,5-PD, panthenol (10%/10%/1%) | DMI, 1,5-PD, panthenol (10%/10%/1%) | - | - | - |
| 10 | 135 ± 4 | 100 ± 8 | 123 ± 7 | 100 ± 7 | 114 ± 3 |
| 30 | 125 ± 9 | 100 ± 5 | 127 ±6 | 100 ± 4 | 103 ±6 |
| 120 | 118 ± 4 | 100 ± 6 | 99 ± 6 | 100 ± 7 | 100 ± 6 |
| 240 | 123 ± 8 | 100 ± 7 | 98 ± 6 | 100 ± 6 | n.d. |

The internal standard (1% Tocopherol in a solution EtOH/octandodecanyl/water) showed the expected values of SAP increase after 10 minutes application.

Emulsion 1, with 2 % resveratrol and the solubiliser mix, showed an immediate increase of the antioxidative power after a 10 minute penetration time and the SAP values remained significantly enhanced over the observed period (240 minutes).

The emulsion 2, with 2% resveratrol but no solubiliser mix increased the SAP values significantly after 10 min and 30 min. However, at longer penetration times the emulsion without the solubiliser mix did not have any influence on the SAP values.

These results clearly show that the presence of resveratrol in conjunction with a solubiliser mix according to the invention has a significant effect on the penetration kinetics of the polyphenol. This effect can most likely be attributed to the higher solubility of resveratrol in the emulsion due to the presence of the solubiliser mix despite the presence of only a low amount of each individual solvent.

Example 6

**The solubiliser mix according to the invention leads to better protection against UV-induced free radicals in the skin.**

The protection provided by a topically applied formulation against UV-induced free radicals inside the skin can be expressed as the SAR value. The SAR value is expressed as a percentage and is obtained based on SAP measurements described in example 5. The difference between the two methods is that in the SAR method, the skin is in addition exposed to UV radiations, which induce free radicals inside the epidermal and dermal layers. These free radicals are neutralized by the antioxidative systems of the skin. In our assay, the remaining SAP value for placebo-treated skin biopsies after exposition to UV irradiation was only 59 % compared to the skin biopsies that were not irradiated. This indicates that the UV irradiation did induce free radical formation and deplete the intrinsic antioxidative system of the skin.

The SAR values for the experiment were measured as follows. The same emulsions as in example 5 (except the 1% tocopherol control) were again applied to the pig skin biopsies, and were left to penetrate for 30 minutes, and were then irradiated with UV radiations (UV solar simulator 300 W Oriel (Newport). E (UVB=280-320nm) = 23,5 W/m2 and E (UVA = 320-400nm) = 180 W/m2). The SAP values were determined before and after UV irradiation and the SAR values were calculated according to the formula:

$$SAR\ [\%] = \frac{SAP\ (Emulsion\ with\ UV) - SAP\ (Placebo\ with\ UV)}{SAP\ (placebo\ without\ UV) - SAP\ (Placebo\ with\ UV)}$$

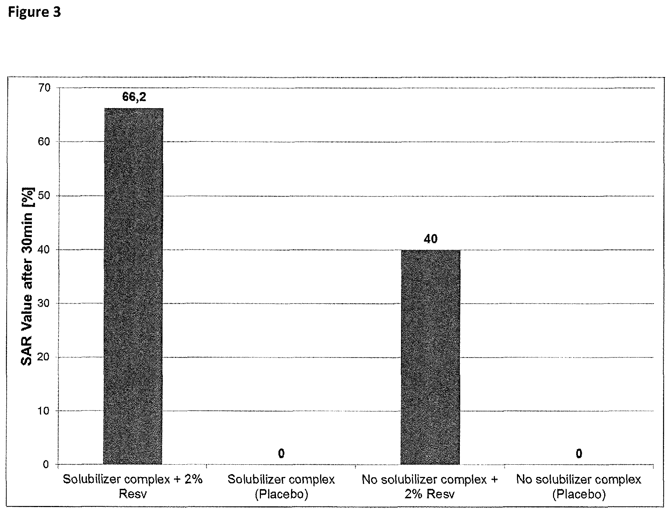

The results of this assay are shown in figure 3.

A SAR value of 0 % means that there is no protective effect of the emulsions applied to the skin. Accordingly, the placebo formulations showed no effect on the SAR value and did not vary, indicating that the basic formulation did not alter the skin's barrier function toward UV irradiation. In contrast, the SAR value for Emulsion 1 is 66 %. This emulsion contains 2 % resveratrol and a solubiliser mix according to the invention and as evident from the high SAR value, has a high protective effect of the skin against UV-irradiation induced free radicals.

Emulsion 2, which contains 2 % resveratrol but no solubiliser mix, also increased the SAP values of the UV-irradiated skin. However, with a SAR value of 40 %, the protective effect of this emulsion is markedly lower as the one of Emulsion 1.

These results indicated that the solubiliser mix improves the penetration of the antioxidant resveratrol significantly, which leads to an increased antioxidative effect even after UV stress. This can likely also be attributed the higher solubility of the polyphenol in the composition.

**FIGURE CAPTIONS**

**Figure 1**

**Antioxidative power measurement of different emulsions by electron spin resonance spectroscopy (ESR).** The figure shows the antioxidative power of a resveratrol-containing emulsions either with or without a solubiliser mix. One of the emulsions is an emulsion without a solubiliser mix that was lyophilised and subsequently reconstituted with solubiliser mix.

**Figure 2**

**Measurement of skin antioxidative potential by electron spin resonance spectroscopy (ESR).** The time-course measurements for two emulsions according to the invention are shown in the figure. The difference between the two emulsions is that one of them (emulsion 1 in table 5) comprises a solubiliser mix (10 wt% dimethyl isosorbide, 10 wt% 1,5-pentandiol and 1 wt% panthenol) and the other (emulsion 2 in table 5) does not.

**Figure 3**

**Skin antioxidative retention experiments.** The SAR values for pig skin biopsies treated with different emulsions with or without resveratrol and with or without the solubiliser mix are shown in this figure.

**Claims**

1. A composition suitable for topical application comprising water, dimethyl isosorbide, a polyol, panthenol or dipotassium glycyrrhizinate and a phenolic or polyphenolic antioxidant, wherein the polyol is 1,5-pentanediol.

2. The composition according to claim 1, wherein the dimethyl isosorbide content is 0.1 to 50 wt%, the polyol content is 0.1 to 50 wt%, and the content of the phenolic or polyphenolic antioxidant is 0.001 to 20 wt%.

3. The composition according to any of the preceding claims, wherein the composition additionally comprises a supporting anti skin irritation compound selected from the group comprising allantoin, ectoin, bisabolol, and/or a plant extract selected from the group comprising Aloe Barbadensis leaf extract and Oriza Sativa bran extract.

4. The composition according to any of the preceding claims, wherein the composition in addition comprises an oil phase.

5. The composition according to claim 4, wherein the composition is an emulsion.

6. The composition of any of the previous claims, wherein the polyol is an alkane diol, glycerol, polyglycerol, a sugar or a sugar derivative.

7. The composition according to any of the preceding claims, wherein the phenolic or polyphenolic antioxidant is a non-flavonoid phenolic or polyphenolic antioxidant, preferably selected from the group consisting of resveratrol and ferulic acid.

8. The composition of any of the preceding claims, wherein the composition in addition comprises at least one further labile active substance selected from the group comprising a further phenolic or polyphenolic antioxidant, ascorbic acid and its derivatives, tocopherol and its derivatives, retinol or retinoic acid derivatives and enzymes such as superoxide dismutase.

9. A method for producing a composition according to any of the preceding claims comprising the steps of:

   a) providing an aqueous solution comprising dimethyl isosorbide, panthenol or dipotassium glycyrrhizinate and a polyol, wherein the polyol is 1,5-pentanediol
   b) providing a phenolic or polyphenolic antioxidant
   c) mixing the aqueous solution and the phenolic or polyphenolic antioxidant shortly before use.

10. The method of claim 9, wherein the phenolic or polyphenolic antioxidant is provided in the form of a lyophilisate.

11. A kit for making a composition according to any of claims 1 to 8 comprising:

   a) an aqueous solution comprising dimethyl isosorbide, panthenol or dipotassium glycyrrhizinate and a polyol, wherein the polyol is 1,5-pentanediol
   b) a phenolic or polyphenolic antioxidant.

12. The kit according to claim 11, wherein the phenolic or polyphenolic antioxidant is provided in the form of a lyophilisate.

13. The kit according to claim 12, wherein the lyophilisate in addition comprises at least one compound selected from the group comprising an additional labile active substance, an oil phase, an emulsifier, and a polymer.

14. Non-therapeutic use of a composition according to any of claim 1 to 8 for topical application.


**Patentansprüche**

1. Eine zur topischen Anwendung geeignete Zusammensetzung umfassend Wasser, Dimethylisosorbid, ein Polyol, Panthenol oder Dikaliumglycyrrhizinat und ein phenolisches oder polyphenolisches Antioxidans, wobei das Polyol 1,5-Pentandiol ist.

2. Die Zusammensetzung nach Anspruch 1, wobei der Dimethylisosorbidgehalt 0,1 bis 50 Gew.%, der Polyolgehalt 0,1 bis 50 Gew.%, und der Gehalt an dem phenolischen oder polyphenolischen Antioxidans 0,001 bis 20 Gew.% beträgt.

3. Die Zusammensetzung nach einem der vorhergehenden Ansprüche, wobei die Zusammensetzung zusätzlich eine unterstützende Anti-Hautirritationsverbindung umfasst, ausgewählt aus der Gruppe umfassend Allantoin, Ectoin, Bisabolol und/oder einen Pflanzenextrakt ausgewählt aus der Gruppe, die Aloe Barbadensis-Blattextrakt und Oriza Sativa-Kleieextrakt umfasst.

4. Die Zusammensetzung nach einem der vorhergehenden Ansprüche, wobei die Zusammensetzung zusätzlich eine Ölphase umfasst.

5. Die Zusammensetzung nach Anspruch 4, wobei die Zusammensetzung eine Emulsion ist.

6. Die Zusammensetzung nach einem der vorhergehenden Ansprüche, wobei das Polyol ein Alkandiol, Glycerin, Polyglycerin, ein Zucker oder ein Zuckerderivat ist.

7. Die Zusammensetzung nach einem der vorhergehenden Ansprüche, wobei das phenolische oder polyphenolische Antioxidans ein nicht-flavonoides phenolisches oder polyphenolisches Antioxidans ist, vorzugsweise ausgewählt aus der Gruppe bestehend aus Resveratrol und Ferulasäure.

8. Die Zusammensetzung nach einem der vorhergehenden Ansprüche, wobei die Zusammensetzung zusätzlich mindestens einen weiteren labilen Wirkstoff umfasst, ausgewählt aus der Gruppe umfassend ein weiteres phenolisches oder polyphenolisches Antioxidans, Ascorbinsäure und ihre Derivate, Tocopherol und seine Derivate, Retinol oder Retinsäurederivate und Enzyme wie Superoxiddismutase.

9. Ein Verfahren zur Herstellung einer Zusammensetzung nach einem der vorhergehenden Ansprüche, umfassend die Schritte:

   a) Bereitstellen einer wässrigen Lösung, umfassend Dimethylisosorbid, Panthenol oder Dikaliumglycyrrhizinat und ein Polyol, wobei das Polyol 1,5-Pentandiol ist
   b) Bereitstellen eines phenolischen oder polyphenolischen Antioxidans
   c) Mischen der wässrigen Lösung und des phenolischen oder polyphenolischen Antioxidans kurz vor dem Gebrauch.

10. Das Verfahren nach Anspruch 9, wobei das phenolische oder polyphenolische Antioxidans in Form eines Lyophilisats bereitgestellt wird.

11. Ein Kit zur Herstellung einer Zusammensetzung nach einem der Ansprüche 1 bis 8 umfassend:

   a) eine wässrige Lösung umfassend Dimethylisosorbid, Panthenol oder Dikaliumglycyrrhizinat und ein Polyol, wobei das Polyol 1,5-Pentandiol ist
   b) ein phenolisches oder polyphenolisches Antioxidans.

12. Das Kit nach Anspruch 11, wobei das phenolische oder polyphenolische Antioxidans in Form eines Lyophilisats bereitgestellt wird.

13. Das Kit nach Anspruch 12, wobei das Lyophilisat zusätzlich mindestens eine Verbindung umfasst ausgewählt aus der Gruppe, die einen zusätzlichen labilen Wirkstoff, eine Ölphase, einen Emulgator, und ein Polymer umfasst.

14. Nichttherapeutische Verwendung einer Zusammensetzung nach einem der Ansprüche 1 bis 8 zur topischen Anwendung.


**Revendications**

1. Composition adaptée pour application topique comprenant de l'eau, l'isosorbide diméthyle, un polyol, du panthénol ou du glycyrrhizinate di-potassium et un antioxydant phénolique ou polyphénolique, dans laquelle le polyol est le 1,5-pentanediol.

2. Composition selon la revendication 1 dans laquelle la proportion d'isosorbide diméthyle est de 0,1 à 50% en masse, la proportion de polyol est de 0,1 à 50% en masse, et la proportion de l'antioxydant phénolique ou polyphénolique est de 0,001 à 20% en masse.

3. Composition selon l'une quelconque des revendications précédentes dans laquelle la composition comprend additionnellement un composé anti-irritation cutanée de soutien choisi dans le groupe constitué par l'allantoïne, l'ectoïne, le bisabolol, et/ou un extrait de plante choisi dans groupe constitué par des extraits de feuilles de Aloe Barbadensis et un extrait de son d'Oriza Sativa.

4. Composition selon l'une quelconque des revendications précédentes dans laquelle la composition comprend en addition une phase huileuse.

5. Composition selon la revendication4 où la composition est une émulsion.

**6.** Composition selon l'une quelconque des revendications précédentes dans laquelle le polyol est un alcane diol, le glycérol, le polyglycérol, un sucre ou un dérivé de sucre.

**7.** Composition selon l'une quelconque des revendications précédentes dans laquelle l'antioxydant phénolique ou polyphénolique est un antioxydant phénolique ou polyphénolique non-flavonoïde, sélectionné préférablement dans le groupe constitué par la resvératrol et l'acide férulique.

**8.** Composition selon l'une quelconque des revendications précédentes dans laquelle la composition comprend en addition au moins une substance labile active supplémentaire sélectionnée dans le groupe constitué par un autre antioxydant phénolique ou polyphénolique, l'acide ascorbique et ses dérivés, le tocophérol et ses dérivés, des dérivés du rétinol ou de l'acide rétinoïque et des enzymes tels le superoxyde dismutase.

**9.** Méthode de préparation d'une composition selon l'une quelconque des revendications précédentes comprenant les étapes suivantes :

a) préparation d'une solution aqueuse comprenant l'isosorbide diméthyle, le panthénol ou le di-potassium glycyrhizinate et un polyol, où le polyol est le 1,5-pentanediol,
b) préparation d'un antioxydant phénolique ou polyphénolique,
c) le mélange de la solution aqueuse et de l'antioxydant phénolique ou polyphénolique juste avant usage.

**10.** Méthode selon la revendication 9 où l'antioxydant phénolique ou polyphénolique est utilisé sous la forme d'un lyophilisat.

**11.** Kit de préparation d'une composition selon l'une quelconque des revendications 1 à 8 comprenant :

a) une solution aqueuse comprenant l'isosorbide diméthyle, le panthénol ou le di-potassium glycyrhizinate et un polyol, où le polyol est le 1,5-pentanediol,
b) un antioxydant phénolique ou polyphénolique.

**12.** Kit selon la revendication 11 où l'antioxydant phénolique ou polyphénolique est utilisé sous la forme d'un lyophilisat.

**13.** Kit selon la revendication 12 où le lyophilisat comprend en addition au moins un composé sélectionné dans le groupe constitué par une substance active labile, une phase huileuse, un émulsifiant et un polymère.

**14.** Composition à usage non thérapeutique selon l'une quelconque des revendications 1 à 8 pour une application topique.

Figure 1

Figure 2

**Figure 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020173472 A

- WO 2009129627 A

**Non-patent literature cited in the description**

- **MANACH et al.** *Am J Clin Nutr May,* 2004, vol. 79 (5), 727-747
- **JUNG K ; RICHTER J ; KABRODT K ; LUCKE IM ; SCHELLENBERG I ; HERRLINGT.** The antioxidative power AP--A new quantitative time dependent (2D) parameter for the determination of the antioxidant capacity and reactivity of different plants. *Spectrochim Acta A Mol Biomol Spectrosc,* 2006, vol. 63, 846-50

- **JUNG K ; SACHER M ; BLUME G ; JANßEN F ; HERRLING T.** How Active are Biocosmetic Ingredients?. *SOFW-Journal,* 2007, vol. 133, 1-2